# EUROPEAN PATENT SPECIFICATION

(11) **EP 0 968 429 B1**
(45) Date of publication and mention of the grant of the patent: **11.12.2002**
(21) Application number: 98906762.4
(22) Date of filing: 19.02.1998
(51) Int. Cl.: G01N 33/68

(54) **METHOD AND KIT FOR DETERMINING THE PHENOTYPE OF A HAPTOGLOBIN AND USE THEREOF**
METHODE UND TESTKIT ZUR BESTIMMUNG DES PHENOTYPS EINES HAPTOGLOBINS UND ANWENDUNG
METHODE ET NECESSAIRE CORRESPONDANT PERMETTANT DE DETERMINER LE PHENOTYPE D'UNE HAPTOGLOBINE ET APPLICATIONS DE CELLE-CI

(30) Priority: 19.02.1997 BE 9700152
(43) Date of publication of application: 05.01.2000
(73) Proprietor: Dade Behring Marburg GmbH, 35001 Marburg (DE)
(72) Inventor: DELANGHE, Joris, B-9300 Aalst (BE); LANGLOIS, Michel, B-9000 Gent (BE); DE BUYZERE, Marc, B-8000 Brugge (BE)
(86) International application number: BE9800023
(87) International publication number: WO98037419

(56) References cited:
- WO-A-90/08324
- US-A- 5 552 295
- CHEMICAL ABSTRACTS, vol. 124, no. 15, 9 April 1996 Columbus, Ohio, US; abstract no. 200157, L. WAGNER ET AL: "Haptoglobin phenotyping by newly developed monoclonal antibodies. Demonstration of haptoglobin uptake into peripheral blood neutrophils and monocytes." XP002071450 & J. IMMUNOL., vol. 156, no. 5, 1996, pages 1989-1996,
- CHEMICAL ABSTRACTS, vol. 85, no. 23, 6 December 1976 Columbus, Ohio, US; abstract no. 173693, J. M. RAMAKERS & H. J. H. KREUTZER: "Turbidimetric determination of haptoglobin" XP002071451 & J. CLIN. CHEM. CLIN. BIOCHEM., vol. 14, no. 8, 1976, pages 407-410,
- DATABASE WPI Section Ch, Week 9422 Derwent Publications Ltd., London, GB; Class B04, AN 94-181949 XP002071452 & SU 1 805 388 A (KIRG CARDIOLOGY RES INST)
- DATABASE WPI Section Ch, Week 9420 Derwent Publications Ltd., London, GB; Class B04, AN 94-165319 XP002071453 & SU 1 800 365 A (A MED RADIATION MED SCI CENTRE)

## Description

This invention relates to a method and a kit for determining the phenotype of a haptoglobin, in particular a human haptoglobin, in a biological fluid.

Human haptoglobins (Hp) have been well studied; they were discovered over 40 years ago and they fulfil many biological key roles such as plasma transport of haemoglobin and interactions with the immune system. All haptoglobins contain two classes of polypeptide chains, beta chains and alpha chains. Beta chains are identical in all haptoglobins, whereas the alpha chains show two forms giving rise to three Hp phenotypes: Hp 1-1, Hp 2-1, Hp 2-2.

Many functional differences exist between the Hp phenotypes, which appear to have important clinical consequences (Langlois & Delanghe, Clin Chem 1996;42:1589-1600). The Hp 2-2 type is characterized by a lower haemoglobin-binding capacity and hence a less efficient protection against oxidative stress, a higher degree of nitric oxide (NO) inhibition, and a less pronounced inhibition of prostaglandin synthesis (Langlois & Delanghe, Clin Chem 1996;42:1589). Furthermore, haptoglobin has angiogenic effects (Hp 2-2 being more angiogenic) (Cid MC et al., J Clin Invest 1993;91:977), agglutinates streptococci by binding to the T4 antigen (Nature 1978;271:373), and is involved in homing and trafficking of leukocytes (Hp binds to the leukocyte adhesion molecules CD11b/CD18 and CD22) (J Biol Chem 1995;270:7543, J Immunol 1996;156:2542). The fact that haptoglobins bind to T4 antigens has already been used for developing an ELISA test for quantitation of human haptoglobin (Katnic I. et al, Arch. Immunol. Ther. Exp. Warsz 1993;42:105-9). This test however does not enable to make a distinction between the different haptoglobin phenotypes since the binding site of the T4 antigen on haptoglobin is the same for the different haptoglobins.

The above described functional properties have important clinical consequences. The Hp 2-2 type is an independent risk factor in refractory essential hypertension and is a predictive marker for target organ damage in essential hypertension (J Hypertens 1993,11:861, J Cardiovasc Risk 1995;2:131). Also in acute myocardial infarction (N Engl J Med 1982;307:457 ) and in coronary atherosclerosis (Atherosclerosis 1997;132:215), the Hp 2-2 phenotype was found to be an important independent risk factor. After correction of the more classical cardiovascular risk factors (e.g. smoking, cholesterol, hypertension,...), the cardiologist remains unable to predict which patient will accumulate atherosclerotic lesions more rapidly. Therefore, knowledge of a genetic predispondence (e.g. Hp type) leads to a better prevention and a more careful follow-up of the cardiovascular patient.

The Hp 2-2 type is associated with a higher mortality of HIV-infection (Trop Med Int Health 1997;11:1102). Kaplan-Meier survival analyis showed a median survival time of approximately 7 years for Hp 2-2 patients versus 11 years for patients carrying another Hp type. This has been explained by a less efficient protection against iron-driven oxidative stress, resulting in a higher viral replication rate. This enhanced viral replication increases the risk for developing therapy-resistant HIV strains, as can be observed in patients with therapeutic resistance towards the potent protease inhibitors. Therefore, knowledge of the patients' Hp type predicts the prognosis of HIV infection and leads to better tailored therapeutic strategies.

Another clinical application can be found in the management of the patient following liver transplantation. Since haptoglobin is exclusively produced by the liver tissue, liver transplantation is a biologically unique condition in which the Hp type of a patient changes (if different from the pre-transplant Hp type). The patients' Hp type in serum exclusively will depend on the haptoglobin phenotype produced by the liver graft.

In a series of 177 patients who underwent a liver transplantation, it became clear that for transplantations with a primary cause of viral hepatitis (n = 88), survival of patients who have recieved a graft expressing the Hp 2-2 phenotype were characterized by a poorer prognosis (P<0.03) as compared with liver transplanted patients who have received a Hp 2-1 or an Hp 1-1 producing graft. As the time frame for controlling the quality of the graft prior to transplantation is very short, a fast Hp phenotyping method could therefore contribute to a better outcome in liver transplantation.

A rapid Hp typing method is useful for paternity testing in forensic medicine. Furthermore, the Hp polymorphism has a genetic influence on the reference values of a broad range of laboratory parameters, not only on the serum Hp concentration (lower for the Hp 2-2 phenotype) but also on plasma lipids (total and LDL-cholesterol), plasma proteins (ferritin, immunoglobulin A, ceruloplasmin), and other biochemical parameters (serum iron, transferrin saturation, vitamin C, vitamin E) (Langlois & Delanghe, Clin Chem 1996;42:1589, Langlois et al, Clin Chem 1996;42:1722). For instance, slight hemolysis in a Hp 2-2 patient can often be confused with a congenital Hp deficiency.

Also in hematology, reference values for peripheral blood B-lymphocytes and CD4+ T-lymphocytes are dependent on Hp type (Langlois et al; Eur J Clin Chem Clin Biochem 1997;35:199). This may lead to an over- or underestimation of CD4+ cell counts when using the fixed CDC classification in the diagnosis of AIDS. Therefore, additional determination of Hp type using a rapid an simple test is helpful to avoid some problems in laboratory medicine.

In summary, the determination of Hp type is an interesting additional test for the clinicians' diagnosis and in the prophylactic and therapeutic management of the patient.

Upto now, haptoglobin phenotyping in serum or plasma is usually performed by starch gel electrophoresis (O. Smithies; Biochemical Journal 1955, 61: 629-41). A drawback of this known technique is that it is slow, laborious and requires the use of electrophoresis equipment.

An object of the present invention is to provide a new method for phenotyping haptoglobins in a biological fluid which is much faster and easier to perform.

To this end, the method according to the invention comprises the steps of :
a) contacting the biological fluid with a haptoglobin binding partner which has at least two locations by which it can bind to haptoglobin so as to be able to agglutinate haptoglobins and/or to be agglutinated thereby to different degrees, depending on the haptoglobin phenotype; measuring the degree of agglutination ;
b) measuring the concentration of the haptoglobin in the biological fluid; and
c) determining based on the degree of agglutination and the haptoglobin concentration the phenotype of the haptoglobin in the biological fluid.

The degree of agglutination can be determined visually or by means of conventional techniques such as turbidimetry and/or nephelometry. In view of the higher accuracy of nephelometry, a particular preference is given to this latter technique although other techniques may also be suitable.

As biological fluid, a sample of all kinds of different biological fluids can be taken. Examples of such biological fluids are blood, plasma, serum, liquor, urine, cell extracts or tissue extracts.

The haptoglobin binding partner which is used in the method according to the invention may be of different types and dimensions. Essential for the binding partner is that it has at least two locations by which it can bind to haptoglobin so as to be able to agglutinate haptoglobins and/or to be agglutinated thereby and this to different degrees, depending on the haptoglobin phenotype.

The locations by which said binding partner can bind to haptoglobins may be formed by a peptide, an antibody, an F_{ab} or F_{ab'} or F(ab')₂ fragment of an antibody, a lectin, a cell receptor, a molecular imprint of a haptoglobin, a bacterial antigen, and/or fragments thereof containing the respective haptoglobin binding site. As bacterial antigen, especially the T4 antigen of Streptococcus pyogenes appeared to be very suited.

The different degree of agglutination can be based on the specificity of the haptoglobin binding location itself. A specific binding location may comprise for example the Fa, F_{ab}, or F(ab')₂ fragment, or even a smaller portion containing the antigen binding site, of an antibody which specifically binds the α2 chain of a haptoglobin of phenotype Hp 2-1 or HP 2-2 but not an α1 chain. The α2 chain is the result of a mutation based on an unequal crossing-over. It comprises 142 amino acids whilst the α1 chain comprises only 83 amino acids. Immunologically, both chains comprise mainly the same epitopes, only in the crossing-over region, the α2 chain has a unique sequence of amino acids. This unique sequence comprises the following sequence of amino acids : ala val gly asp lys leu pro glu cys glu ala asp asp gly gin pro pro pro lys cys ile. Suitable epitopes for making selective antibodies can thus be selected within this sequence. In order to be selective, the epitopes have to comprise at least the portion "glu ala asp" from the previous sequence.

For making specific antibodies, synthetic peptides corresponding to the selected epitopes can first of all be made. Such synthetic peptides capable of raising haptoglobin-specific antibodies have to be coupled to an immunogenic protein (carrier protein) e.g. keyhole limpet hemocyanin, bovine serum albumin and the like. Lines of somatic cells immunised against the synthetic peptides can be obtained by immunisation of suited animals e.g. mice (e.g. BALB/c), rabbits, sheeps, goats, etc. The preparation of monoclonal antibodies could be done e.g. using the method of Fazekas, de St. Groth S. and Scheidegger, D., "Production of monoclonal antibodies: strategy and tactics", J. Immunol. Meth., 35, 1, 1980,

In general, the host (mice, rabbits, sheeps, goats, etc.) is immunised by administering the peptide-protein conjugate using any suitable injection method, either intraperitoneally, intravenously, subcutaneously, etc. Suitable adjuvants (e.g. Freund's) may be included in the immunisation protocol.

The initial immunisation with the antigen is normally followed by several booster injections given periodically at intervals of several weeks. In case of monoclonals the immunised somatic cells, preferably spleen cells, must then be fused with a myeloma cell line (e.g. SP2/0) to produce hybridomas capable of secreting specific antibodies.

The detection of specific antibodies (hybridomas or immune serum) may be performed by any suitable assay such as enzyme immunoassays, radioimmunoassays and/or nephelometric or turbidimetric assays. Appropriate screening procedures comprise the selection of specific antibodies on their binding abilities to the synthetic peptides, to the peptide-protein conjugates, to the carrier proteins and to the different haptoglobins phenotypes. Those antibodies are preferred which bind specifically to the synthetic peptides, to the peptide portion of the peptide protein conjugates and to haptoglobin Hp 2-2 and/or 2-1.

Maintenance of the hybridomas is accomplished by the use of appropriate standard tissue culture media containing preferably foetal calf serum.

Production of higher yields of monoclonals could be conducted via ascites or serum free mass cell culture (roller bottles, fermenter, etc.).

How to isolate the desired antibodies (from culture supernatant, ascites or serum) is a matter of routine art. Well known techniques are salt precipitation, gel chromatography, ion exchange chromatography, affinity chromatography (e.g. Protein A) and the like.

Antibodies which are specific to the α2 chains can be used as such as haptoglobin binding partners for agglutinating haptoglobins of phenotype Hp 2-2 and 2-1. Due to the fact that the Hp 2-2 phenotype comprises only α2 chains whilst the Hp 2-1 phenotype comprises α1 and α2 chains, a difference in agglutination degree can be observed.

In the above described embodiment, the binding partner is formed by a molecule, namely by an antibody, which is able to form a lattice or network with the haptoglobin molecules and which is thus capable of agglutinating them. Instead of natural molecules, it is also possible to develop artificial molecules which are able to agglutinate haptoglobins. This can be done by directly (chemically) or indirectly (e.g. using biotin and avidin or streptavidin) coupling for example haptoglobin binding molecules such as antibodies, antibody fragments, eucaryotic and/or procaryotic cellular receptors or parts thereof to other molecules, possibly in combination with other haptoglobin binding molecules which are specific or non-specific for the different haptoglobin phenotypes, for example a T4 antigen or the binding portion thereof.

In a variant embodiment, the antibodies, in particular polyclonal or monoclonal antibodies, or fragments thereof containing the haptoglobin binding sites, such as F_{ab^{'}} F_{ab}, or F(ab')₂ fractions or even smaller fractions, can be adhered to carrier particles, in particular to synthetic or mineral particles but also to natural cells or cell fragments. In this way, the sensitivity of the assay can be improved. The particles have such dimensions that the network formed thereby, or in other words the degree of agglutination, can easily be determined e.g. turbidimetrically and/or nephelometrically. The dimensions of the carrier particles are preferably comprised between 0.02µ and 2µ, and most preferably between 0.1µ and 0.8µ, in view of improving the sensitivity of the assay.

Among the carriers which can be employed are latex particles for example based on polysterene, polymethacrylate, polyacrylate, polyvinylacetacrylate, polyvinylpyridine, vinylchloridacrylate, polybutadienestyrene or polybutadieneacrylonitrilstyrene copolymers. Useful might be as well phenolic resins or finely divided cellulose or amino cellulose particles. The carriers used can be further erythrocytes, the substances bentonite, cholesterol crystals, quartz or micro particles from inorganic oxides, like silicium dioxide, alumina oxide or other finely dispersed minerals. Disperses metals as for instance gold, silver or others might be used as well. Useful might be further dispersed bacteria as staphylococcus or streptococcus, bacillus prodigious, rickethsia or cell membrane fragments.

The binding of antigens or antibodies to the carriers could be accomplished by adsorption or covalent techniques.
Examples for a covalent binding are
a) the aldehyde method given in the US patent 4,448,908;
b) the carbodimide method according to Gross et al,: Immunochemistry, Vol. 5, p. 55, 1968;
c) the acid chloride method and acid anhydride method according to Erlanger et al.: Journal of Biological Chemistry Vol. 228, p. 713, 1957;
d) the isocyanate method - Goodfriend et al.: Canadian Journal of Biochemistry and Physiology, Vol. 36, p. 1171, 1958.

According to the invention, it has been found that, in order to obtain a measurable difference in degree of agglutination between the different haptoglobin phenotypes, the locations on the binding partners by which the haptoglobins are bound do not have to be specific for the different haptoglobin phenotypes. It is presumed that the different degrees of agglutination can be attributed to the different molecular weights of the haptoglobin phenotypes, in other words to their different sizes, and to the different contents of α₁, α₂ and β chains. The average molecular weight decreases more particularly from the Hp 2-2 phenotype, over the Hp 2-1 phenotype to the Hp 1-1 phenotype.

Tests have been carried out with bacterial strains, in particular with Streptococcus pyogenes, which carry the T4 antigen. This antigen binds to the β chain of haptoglobins. Although the binding itself is not specific, the degree of agglutination is sufficiently different to make a distinction between the different haptoglobin phenotypes, in particular also between the Hp 2-1 and Hp 2-2 phenotypes. In this respect, it should be noted that the fact that Streptococcus pyogenes carrying T4 antigens can be agglutinated by Hp 2-2 and Hp 2-1 but not by Hp 1-1 was already disclosed in Nature 1978; 271:373. However from this publication it is not clear that, based on this property, an agglutination assay can be developed for making a distinction between the different haptoglobin phenotypes, especially not for making a distinction between the Hp 2-1 and Hp 2-2 phenotypes.

Instead of using entire bacteria, one could also consider using fragments thereof carrying the bacterial antigen which forms the haptoglobin binding location. Use could be made for example of a bacterial lysate.

Beside procaryotic bacterial cells, also some eucaryotic cells or fractions thereof showing haptoglobin receptors may be suited for obtaining the desired agglutination reaction. Suitable receptors are in particular CD receptors, more particularly CD22 receptors. These receptors may be located on animal or human cells, in particular on leukocytes.

According to the invention, the haptoglobin binding locations do not necessarily have to be situated naturally on the carrier particles but, as explained hereinabove in relation to the specific antibodies, it is possible to attach the haptoglobin binding fragments by adsorption and/or covalent binding to other carriers, in particular to synthetic or mineral carrier particles or to natural cells or fragments thereof. In case the binding fragments are not specific for different haptoglobin phenotypes, the carrier particles have preferably a diameter of between 0.5 µ and 4 µ, and more preferably of between 1 µ and 3 µ. These diameters are indeed not only important for the determination of the degree of agglutination by turbidimetry or nephelometry, but it has now been found that the dimensions of the carrier particles play also a role in the different agglutinating properties of the haptoglobin phenotypes. The dimensions of the carrier particles are more particularly chosen such that the relatively small haptoglobin molecules of the Hp 1-1 phenotype are not or nearly not able to agglutinate the carrier particles whilst the larger molecules of the Hp 2-1 phenotype and the on average still larger molecules of the Hp 2-2 phenotype are able to agglutinate them and this more particularly even to a different degree for the Hp 2-1 and HP 2-2 phenotypes. In a particular embodiment, the carrier particles may additionally be adhered to one another, in particular to produce chains of 5 to 20 particles, similar to the chains formed by Streptococcus pyogenes.

For providing the locations to which the haptoglobins can bind on the carrier particles of the haptoglobin binding partner, use can not only be made of the above described specific antibodies or antibody fragments and the T4 antigen, but further of other substances which bind to haptoglobins. As mentioned already hereinabove, these substances may generally include a peptide, an antibody, an F_{ab}, F_{ab'} or F(ab')₂ fragment of an antibody, a lectin, a cell receptor, a molecular imprint of a haptoglobin, a bacterial antigen, and/or fragments thereof containing the respective haptoglobin binding site. Use can in particular be made of poly- or monoclonal antibodies against haptoglobins which do not have to bind specifically to one of the haptoglobin phenotypes. Moreover, one could consider using for example the leukocyte adhesion molecules (lectins) CD11b/CD18 and/or CD22 as haptoglobin binding sites. At least two binding locations are provided on each carrier particle so that a network or lattice can be formed in the biological fluid, resulting in the intended agglutination.

The present invention still provides a further solution for obtaining the required difference in agglutination degrees. Instead of using carrier particles of a particular size, the haptoglobin binding locations can indeed also be provided on molecules, in particular macromolecules, which are thus able to agglutinate haptoglobins of a different size to a different degree due to the steric hindrances. The degree of agglutination can then be measured after a time sufficient to obtain the final agglutination or sooner so that also the binding kinetics play a role in the measured degree of agglutination.

The degree of agglutination is not only dependent on the haptoglobin phenotype, but also on the concentration of the haptoglobin in the biological fluid. In a preferred embodiment, the concentration of the haptoglobin is therefore also measured and taken into account when determining the phenotype on the basis of the degree of agglutination. Preferably, the relationship between the concentration of a reference haptoglobin in the biological fluid and the degree of agglutination is first determined for at least one reference haptoglobin phenotype, in particular for a haptoglobin phenotype which causes a sufficient degree of agglutination. Normally, this will be the Hp 2-2 or Hp 2-1 phenotype, preference being given to either both of them or to the Hp 2-2 phenotype. The phenotype of the haptoglobin in the biological fluid is then determined by comparing the relationship between the measured concentration of the haptoglobin and the measured degree of agglutination with the same relationship which has first been established for the reference haptoglobin. It has indeed been found that there is a significant difference in agglutination properties, for a same haptoglobin concentration, between the haptoglobins of phenotypes Hp 1-1, Hp 2-1 and Hp 2-2.

In a particularly preferred embodiment of the method according to the present invention, for determining the phenotype of a haptoglobin in a biological fluid, the measured degree of agglutination is correlated by means of the relationship, which has been established between the concentration and degree of agglutination for the reference haptoglobin, to a particular concentration of the reference haptoglobin, namely to that concentration of the reference haptoglobin which provides the same degree of agglutination. Then, the ratio between this concentration and the actually measured concentration is calculated. The thus calculated ratio is indicative of the phenotype of the haptoglobin in the biological fluid. Indeed, when haptoglobin Hp 2-2 has been taken as reference haptoglobin, a calculated value near 1 means of course that the haptoglobin is of the Hp 2-2 phenotype. Experimentally, it has been found that this ratio is further situated near 0.5 in case the haptoglobin is of the Hp 2-1 phenotype and even much smaller, i.e. near 0.125 in case the haptoglobin is of the Hp 1-1 phenotype.

In a particular embodiment of the kit according to the present invention for determining the phenotype of a haptoglobin in a biological fluid is provided for establishing the relationship between the degree of agglutination and the haptoglobin concentration for at least one reference haptoglobin phenotype. Indeed, it comprises, in addition to the above described haptoglobin binding partner, at least one sample containing an haptoglobin of a predetermined phenotype. This sample may be a sample of the same biological fluid, which may for example have been lyophilised or otherwise treated to increase the shelf life thereof, or may be an artificially prepared solution. Moreover, the haptoglobin sample may be a sample of a solid haptoglobin or a substantially pure preparation thereof. The kit may additionally comprise a solution for diluting or dissolving the haptoglobin sample.

By means of this sample the degree of agglutination can be determined for one or more concentrations (dilutions) of the predetermined haptoglobin. From practical tests performed with suspensions of Streptococcus pyogenes as haptoglobin binding partner, it appeared to be important to be able to determine, when performing a test or a series of tests, each time the degree of agglutination for one or more concentrations of the predetermined haptoglobin, or in other words to calibrate the measurement of the degree of agglutination.

The haptoglobin calibration or control sample included in the kit contains preferably a haptoglobin of the Hp 2-2 or Hp 2-1 phenotype, and most preferably a haptoglobin of the Hp 2-2 phenotype, since such phenotype generally causes the highest degree of agglutination.

As mentioned hereinabove, different types of haptoglobin binding partners may be appropriate for obtaining the desired agglutination reaction. First of all, use can be made of bacteria, in particular of Streptococcus pyogenes. In order to increase the shelf life thereof, these bacteria, or fragments thereof, can also be included in the kit in lyophilised form. In view of the shelf life and also in view of obtaining more easily a standardised agglutination reaction, preference may be given to a non-living haptoglobin binding partner. The binding partner may comprise for example carrier particles having the locations to which the haptoglobins can bind artificially attached thereto. The haptoglobin binding partner may in particular comprise an antibody which specifically binds an α2 chain of a haptoglobin of phenotype Hp 2-1 or Hp 2-2 but not an α1 chain or at least the binding site of said specific antibody. As described hereinabove, this antibody is preferably attached to carrier particles. When the agglutination reaction, i.e. the haptoglobin binding partner, can be sufficiently standardised, the phenotyping can be carried out without requiring the use of a sample containing a reference haptoglobin, or in other words the kit for determining the phenotype of a haptoglobin may essentially consist of the haptoglobin binding partner itself.

In a particular embodiment of the kit according to the invention, it further comprises a preparation of antibodies which are immunoreactive with all the different haptoglobin phenotypes to a same degree. Such preparation enables to determine the haptoglobin concentration in the biological fluid, and if still necessary, in the haptoglobin calibration sample, by immunonephelometry.

According to the invention, the above described method and kit for determining the haptoglobin phenotype can be used for different purposes.

First of all, the use of the method or kit according to the invention is proposed for assessing atherosclerotic risk. In case a Hp 2-2 phenotype is found, this is correlated with a higher risk.

The use of the method or kit according to the present invention is further proposed for estimating a patient's prognosis following viral infection, in particular a HIV infection. In this particular use, the determination of a Hp 2-2 haptoglobin phenotype is correlated with a worsened prognosis.

The use of the method or kit according to the invention is moreover proposed for determining graft survival following transplantation, in particular liver transplantation, the determination of a Hp 2-2 haptoglobin phenotype of the transplanted liver being in this case correlated with a lower survival.

The use of the method or kit according to the present invention is finally also proposed for improving the interpretation of laboratory parameters such as serum haptoglobin concentration, plasma lipids, in particular total lipids and LDL-cholesterol, plasma proteins, in particular ferritin, immunoglobulin A and ceruloplasmin, and other biochemical parameters such as serum iron, transferrin saturation, vitamin C and vitamin E. Reference values established for these parameters are indeed also to be correlated with the haptoglobin phenotype.

Further particularities and advantages of the invention will become apparent from the following example of a method according to the invention. This example is, however, not intended to limit the scope of the invention. The results obtained are indicated in the drawings wherein :
Figure 1 shows a typical calibration curve of the agglutination assay performed in Example 1 ; and
Figure 2 shows the relationship between the relative agglutination and the haptoglobin concentration and this for the different haptoglobin phenotypes.

### Example

In this example, the phenotype of the haptoglobin present in different blood serum samples was determined. Use was made as binding partner for the agglutination reaction of bacterial cells, more particularly of freshly grown Streptococcus pyogenes strains carrying the T4 antigen.

### Preparation of the bacterial suspension.

The Streptococcus pyogenes strains had been grown overnight on commercial blood agar media plates (Becton Dickinson, Erembodegem, Belgium) at 37 °C prior to diagnostic use. The colonies formed on the plates were transferred into a 0.9% NaCI solution. This solution was agitated to obtain a homogeneous bacterial suspension. The viscosity of the bacterial suspension was increased by final concentrations of 10% glycerol so that the a stable suspension was obtained. The suspensions were diluted or additional bacteria were added until the absorbance (at a wavelength of 600 nm) in a standard 1 cm-cuvette was equal to about 0.250. The bottle containing the suspension was well agitated before use, at least when there were longer periods between the measurements.

### Measurement of the haptoglobin concentrations.

Haptoglobin concentrations were measured immunonephelometrically using the Behring nephelometer II (BN II) of Behring Diagnostics GmbH, D-35001 Marburg, Germany. The nephelometer was calibrated against the international CRM 470 protein standard (reference: Whicher et al. Clin Chem 1994;40:934-8). The agglutination reaction was obtained by means of the rabbit anti-human haptoglobin antibodies provided in the quantitative haptoglobin determination kit of Behring Diagnostics. Further, the procedure provided in this kit was followed. More information about this procedure can be found in Fink PC et al. J. Clin. Chem. Clin. Biochem. 1989;27;261-76.

### Measurement of the degree of agglutination.

The degrees of agglutination in the different serum samples were also measured by means of the BN II nephelometer. For obtaining the required agglutination, 50 µl of the above bacterial suspension was each time added to 20 µl serum sample.

Calibration of the assay was obtained using a serum pool originating from a healthy blood donor carrying the Hp 2-2 phenotype which was chosen in this example as reference haptoglobin. Haptoglobin concentration of this serum pool was determined as described hereabove and was adjusted by means of a 0.9 % NaCI solution to a concentration of 1 g/l Hp 2-2. This standard was arbitrarily set at 100 agglutination units/g Hp 2-2. Serial dilutions of the serum pool in physiological (0.9 % sodium chloride) saline solution (100%, 40%, 20% of the initial concentration) were made to construct the calibration curve. For constructing this curve, the following program was set on the BN nephelometer :

| | | |
|---|---|---|
| Test No. | 53 | Abbreviation HTYP |
| Test name | HAPTOCOC | |
| Sample volume (µl) | 20 | Sample dil. 1 :1.0 |
| | | Minimal-dilution 1.0 |
| Reagent 1 vol. (µl) | 50 | T4 |
| Reagent 2 vol. (µl) | 0 | |
| React. buffer vol(1) | 50 | N Reaction Buffer |
| React. Buffer vol(2) | 50 | |
| Measuring time (min) | 12 | Fixed Time |
| Standard | | pool |
| No.of Std. points | 4 | |
| First dilution | 1 : 1.0 | |
| Deviation allowed (%) | 99.9 | |
| Validity (days) | 7 | |
| Conc. unit | units | |
| Measuring range (units) | | |
| Lower level | 0.00 | |
| Upper level | 1300.0 | |

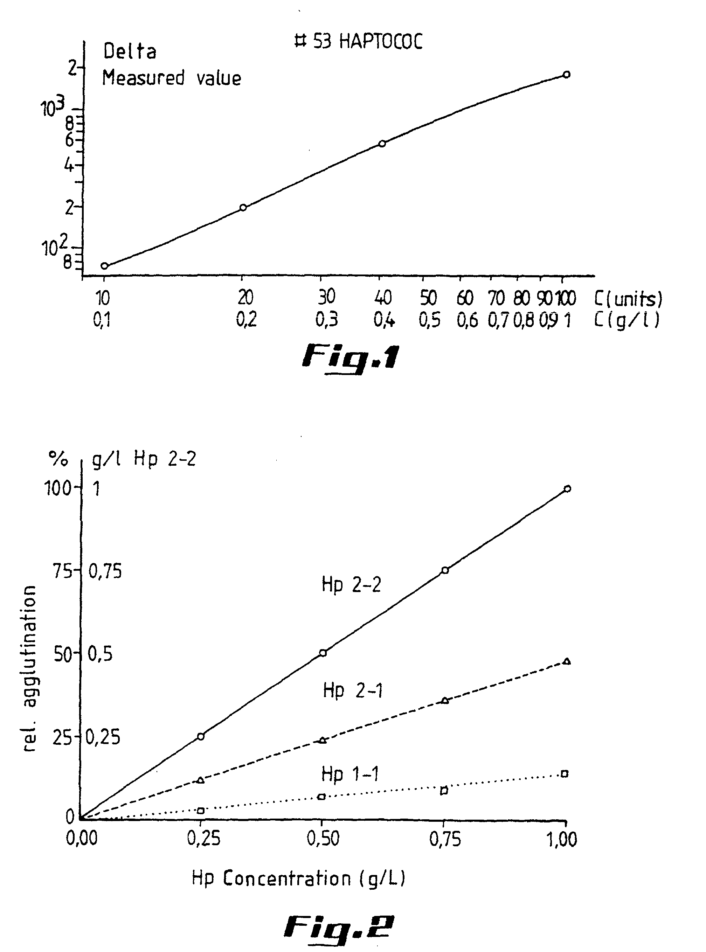

For each of the four concentration, corresponding to 1.0; 0.4 ; 0.2 and 0.1 g Hp 2-2/l, the difference in value measured before and after the addition of the bacterial suspension was calculated and was used to indicate the degree of agglutination. These delta measurement values were respectively 1799; 584; 195 and 74 and are shown in Figure 1. In this Figure, both the agglutination units and the corresponding Hp 2-2 concentrations have been indicated on the X-axis.

With the same nephelometer and the same bacterial suspension, the difference in measured value was again determined for a number of unknown serum samples. The delta measurement values corresponded, according to the graph in Figure 1, to a certain amount of agglutination units or to a particular concentration of Hp 2-2 haptoglobin, namely to a concentration of Hp 2-2 haptoglobin giving the same degree of agglutination.

### Determination of the haptoglobin phenotype.

Before determining the phenotype of a haptoglobin of an unknown phenotype, the degree of agglutination was first of all measured for a large number of serum samples containing the different haptoglobin phenotypes. For each of these samples, the degree of agglutination and the haptoglobin concentration was measured as set forth hereabove. Further, based on the calibration graph shown in Figure 1, or similar calibration graphs established when performing the different measurements, the measured degree of agglutination was correlated to the corresponding Hp 2-2 concentration or, in other words, to the amount of agglutination units (relative agglutination in %). A linear regression analysis was applied to the obtained data about the measured concentrations and the relative agglutination percentages and the calculated regression lines for the different haptoglobin phenotypes were shown in Figure 2. When both on the X and the Y axis, the measured haptoglobin concentration and the relative agglutination is expressed in g/l, the regression coefficients were respectively about 1 for the HP 2-2 phenotype, 0.5 for the Hp 1-1 phenotype and 0.125 for the Hp 1-1 phenotype.

For determining the phenotype of the haptoglobin in unknown serum samples, the haptoglobin concentration of these samples is measured in addition to the degree of agglutination and the corresponding concentration of the reference haptoglobin Hp 2-2. These measurements of the haptoglobin concentration were also done by means of the BN II nephelometer according to the procedure referred to hereabove. Subsequently, the ratio's between the corresponding Hp 2-2 concentrations, determined on the basis of the measured degrees of agglutination, and the actually measured haptoglobin concentrations were calculated. A ratio near 1 indicated that the haptoglobin in the serum sample was of the Hp 2-2 phenotype, a smaller ratio, more particularly a ratio near 0.5 indicated that the haptoglobin in the serum sample was of the Hp 2-1 phenotype, and a very small ratio, namely near 0.12 indicated that the haptoglobin in the serum sample was of the Hp 1-1 phenotype.

From the above example, it can be concluded that, in view of the significant difference between the degrees of agglutination, at a same concentration, for the different haptoglobin phenotypes, the method according to the present invention is a very reliable and fast method for phenotyping haptoglobins.

### REFERENCES

1. M. Langlois, J. Delanghe. Biological and clinical significance of haptoglobin polymorphism in humans Clinical Chemistry 1996;42:1589-1600.
2. D. Bernard, M. Langlois, M. De Buyzere, J. Delanghe. Evolution of haptoglobin concentration in serum during the early phase of acute myocardial infarction. European Journal of Clinical Chemistry and Clinical Biochemistry 1997;35:85-88.
3. M. Langlois, J. Delanghe, J. Philippe, J. Ouyang, D. Bernard, M. De Buyzere, G. Van Nooten, G. Leroux-Roels. Distribution of lymphocyte subsets in peripheral blood and bone marrow is associated with haptoglobin type. Binding of haptoglobin to the B cell lectin CD22. European Journal of Clinical Chemistry and Clinical Biochemistry 1997;35:199-204.
4. M. Langlois, J. Delanghe, M. De Buyzere, D. Bernard, J. Ouyang. Effect of haptoglobin on the metabolism of vitamin C. American Journal of Clinical Nutrition 1997;66:606-610.
5. J. Delanghe, B. Cambier, M. Langlois, M. De Buyzere, H. Neels, D. De Bacquer, Ph. Van Cauwelaert. Haptoglobin polymorphism, a genetic risk factor in coronary artery bypass surgery. Atherosclerosis 1997;132:215-219.

## Claims

1. A method for determining the phenotype of a haptoglobin in a biological fluid, **characterized in that** it comprises the steps of:
a) contacting a sample of the biological fluid with a haptoglobin binding partner which has at least two locations by which it can bind to haptoglobin so as to be able to agglutinate haptoglobins and/or to be agglutinated thereby to different degrees, depending on the haptoglobin phenotype, and measuring the degree of agglutination ;
b) measuring the concentration of the haptoglobin in the biological fluid; and
c) determining, based on the degree of agglutination and the haptoglobin concentration, the phenotype of the haptoglobin in the biological fluid.

2. A method according to claim 1, **characterized in that** the locations by which said binding partner can bind to haptoglobins are formed by a peptide, a monoclonal or polyclonal antibody, an F_{ab}, F_{ab}, or F(ab')₂ fragment of an antibody, a lectin, a cell receptor, a molecular imprint of a haptoglobin, a bacterial antigen, and/or fragments thereof containing the respective haptoglobin binding site.

3. A method according to claim 2, **characterized in that** said locations are formed by a T4 antigen or a haptoglobin binding fragment thereof.

4. A method according to claim 1 or 2, **characterized in that** said locations or the entire haptoglobin binding partner is formed by an antibody which specifically binds an α2 chain of a haptoglobin of phenotype Hp 2-1 or Hp 2-2 but not an α1 chain.

5. A method according to claim 4, **characterized in that** said antibody specifically binds an epitope selected within the following amino acid sequence of an α2 chain: ala val gly asp lys leu pro glu cys glu ala asp asp gly gin pro pro pro lys cys ile and comprising at least the following sequence: glu ala asp.

6. A method according to any one of the claims 1 to 5 **characterized in that** said binding partner consists mainly of a molecule, possibly made by chemical binding of two or more molecules so as to provide said at least two haptoglobin binding locations, which molecule is able to agglutinate haptoglobins of different phenotypes to different degrees.

7. A method according to claim 6, **characterised in that** said binding locations are specific for different haptoglobin phenotypes in order to obtain said different degree of agglutination.

8. A method according to claim 6 or 7, **characterised in that** said molecule has a size such that said different degree of agglutination is obtained.

9. A method according to any one of the claims 1 to 5, **characterized in that** said binding partner comprises carrier particles.

10. A method according to claim 9, **characterized in that** said carrier particles comprise eucaryotic and/or procaryotic cells or a fraction thereof carrying receptors forming said locations to which haptoglobins can bind.

11. A method according to claim 10, **characterized in that** said cells are cells of Streptococcus pyogenes carrying in particular T4 antigens as haptoglobin receptors.

12. A method according to claim 10, **characterized in that** said cells are animal or human cells, in particular leukocytes, carrying CD receptors, in particular CD22 receptors.

13. A method according to claim 9, **characterized in that** said binding partner comprises carrier particles having said locations attached thereon, preferably by adsorption and/or covalent binding, said particles comprising in particular synthetic or mineral particles or natural cells or fragments thereof.

14. A method according to claim 13, **characterized in that**, in case binding locations are specific, said carrier particles have a diameter of between 0.02 and 2 µ, and preferably, in case said binding locations are specific for different haptoglobin phenotypes, of between 0.1 and 0.8 µ, and, in case said binding locations are not specific for different haptoglobin phenotypes, of between 0.5 µ and 4 µ, and more preferably of between 1 µ and 3 µ.

15. A method according to claim 13 or 14, **characterized in that** the carrier particles have bacterial antigens, in particular T4 antigens, haptoglobin antibodies which are specific for the different haptoglobin phenotypes or not, and/or haptoglobin binding fragments of said antigens or antibodies adhered thereto to form said locations by which the binding partner can bind to haptoglobin.

16. A method according to any one of the claims 1 to 15, **characterized in that** the relationship between the concentration of a reference haptoglobin in the biological fluid and the degree of agglutination is first established for at least one reference haptoglobin phenotype, in particular for haptoglobin phenotype Hp 2-1 and/or Hp 2-2, and preferably for haptoglobin phenotype Hp 2-2, and the phenotype of the haptoglobin in the biological fluid is determined by comparing the relationship between the measured concentration of the haptoglobin and the measured degree of agglutination with said established relationship between the concentration of the reference haptoglobin and the degree of agglutination obtained by said reference haptoglobin.

17. A method according to claim 16, **characterized in that** for determining the phenotype of the haptoglobin in the biological fluid, the measured degree of agglutination is correlated by means of said relationship established for the reference haptoglobin to a particular concentration of this reference haptoglobin, the ratio between this particular concentration and the measured haptoglobin concentration is calculated, and based on this ratio the phenotype of the haptoglobin is determined taking into account that, when taking haptoglobin Hp 2-2 as reference haptoglobin, the calculated ratio would be on average near 1; 0.5 and 0.125 respectively in case the haptoglobin in the biological fluid is of phenotype Hp 2-2, Hp 2-1 and Hp 1-1.

18. A method according to any one of the claims 1 to 17, **characterized in that** the degree of agglutination is measured nephelometrically and/or turbidimetrically.

19. A kit for determining the phenotype of a haptoglobin in a biological fluid according to the method of claim 1, **characterized in that** it comprises:
a) a haptoglobin binding partner which has at least two locations by which it can bind to haptoglobin so as to be able to agglutinate haptoglobins haptoglobins and/or to be agglutinated thereby to different degrees, depending on the haptoglobin phenotype; and
b) at least one sample containing an haptoglobin of a predetermined phenotype.

20. A kit according to claim 19, **characterized in that** the haptoglobin in said sample is of the Hp 2-2 or Hp 2-1 phenotype, and preferably of the Hp 2-2 phenotype.

21. A kit according to claim 19 or 20, **characterized in that** it further comprises at least one reagent to measure the concentration of haptoglobins, in particular a preparation of antibodies which bind to the different haptoglobin phenotypes.

22. Use of the method according to any one of the claims 1 to 18 or of the kit according to any one of the claims 19 to 21 for assessing atherosclerotic risk, wherein the determination of a Hp 2-2 haptoglobin phenotype is correlated with a higher atherosclerotic risk than the determination of a Hp 1-1 or 2-1 haptoglobin phenotype.

23. Use of the method according to any one of the claims 1 to 18 or of the kit according to any one of the claims 19 to 21 for estimating a patient's prognosis following viral infection, in particular a HIV infection, wherein the determination of a Hp 2-2 haptoglobin phenotype is correlated with a worsened prognosis than the determination of a Hp 1-1 or 2-1 haptoglobin phenotype.

24. Use of the method according to any one of the claims 1 to 18 or of the kit according to any one of the claims 19 to 21 for determining graft survival following transplantation, in particular liver transplantation, wherein the determination of a Hp 2-2 haptoglobin phenotype is correlated with a lower survival than the determination of a Hp 1-1 or 2-1 haptoglobin phenotype.

25. Use of the method according to any one of the claims 1 to 18 or of the kit according to any one of the claims 19 to 21 for improving the interpretation of laboratory parameters selected from the group comprising serum haptoglobin concentration, plasma lipids, in particular total lipids and LDL-cholesterol, plasma proteins, in particular ferritin, immunoglobulin A and ceruloplasmin, and other biochemical parameters such as serum iron, transferrin saturation, vitamin C and vitamin E, wherein the determination of a Hp 2-2 haptoglobin phenotype is correlated with other reference values than the determination of a Hp 1-1 or 2-1 haptoglobin phenotype.

26. Use of a haptoglobin binding partner, which has at least two locations by which it can bind to haptoglobin so as to be able to agglutinate haptoglobins and/or to be agglutinated thereby to different degrees, depending on the haptoglobin phenotype, for determining the phenotype of a haptoglobin in a biological fluid, in particular in blood, plasma, serum, liquor, urine, cell extract or tissue extract.

27. Use according to claim 26, **characterized in that** the said locations on the haptoglobin binding partner are formed by a peptide, a monoclonal or polyclonal antibody, an F_{ab}, F_{ab'} or F(ab')₂ fragment of an antibody, a lectin, a cell receptor, a molecular imprint of a haptoglobin, a bacterial antigen, and/or fragments thereof containing the respective haptoglobin binding site.

28. Use according to claim 26 or 27, **characterized in that** the haptoglobin binding partner comprises bacterial cells, in particular cells of Streptococcus pyogenes carrying T4 antigens, or fractions thereof containing haptoglobin binding sites.

29. A haptoglobin binding partner for use in a method according to any one of the claims 1 to 18 and/or in the kit according to any one of the claims 19 to 21, **characterized in that** it is different from entire cells of Streptococcus pyogenes and has at least two locations by which it can bind to haptoglobin so as to be able to agglutinate haptoglobins and/or to be agglutinated thereby to different degrees, depending on the haptoglobin phenotype.

30. A haptoglobin binding partner according to claim 29, **characterized in that** it comprises carrier particles having said locations attached thereon, preferably by adsorption and/or covalent binding.

31. A haptoglobin binding partner according to claim 29 or 30, **characterized in that** it comprises an antibody or an antibody fragment which specifically binds an α2 chain of a haptoglobin of phenotype Hp 2-1 or Hp 2-2 but not an α1 chain.

32. A kit for determining the phenotype of a haptoglobin in a biological fluid, **characterized in that** it comprises a haptoglobin binding partner as defined in any one of the claims 29 to 31.

## Patentansprüche

1. Verfahren zur Bestimmung des Phenotyps eines Haptoglobins in einem biologischen Fluid, **dadurch gekennzeichnet, daß** es die Schritte:
a) In-Kontakt-Bringen einer Probe des biologischen Fluids mit einem Haptoglobinbindungspartner, welcher mindestens zwei Stellen besitzt, durch welche er sich an Haptoglobin binden kann, um so fähig zu sein, Haptoglobine zu agglutinieren und/oder dadurch in unterschiedlichem Ausmaß agglutiniert zu werden, in Abhängigkeit vom Haptoglobinphenotyp, und Messen des Ausmaßes der Agglutinierung;
b) Messen der Konzentration des Haptoglobins im biologischen Fluid; und
c) Ermitteln des Phenotyps des Haptoglobins im biologischen Fluid, basierend auf dem Ausmaß der Agglutinierung und der Haptoglobinkonzentration,
umfaßt.

2. Verfahren nach Anspruch 1, **dadurch gekennzeichnet, daß** die Stellen, durch welche sich der genannte Bindungspartner an Haptoglobine binden kann, durch ein Peptid, einen monoklonaien oder polyklonalen Antikörper, ein F_{ab}-, F_{ab'}- oder F(ab')₂-Fragment eines Antikörpers, ein Lectin, einen Zellrezeptor, einen molekularen Imprint eines Haptoglobins, ein bakterielles Antigen und/oder Fragmente hievon, welche die jeweilige Haptoglobinbindungsstelle enthalten, ausgebildet wird.

3. Verfahren nach Anspruch 2, **dadurch gekennzeichnet, daß** die genannten Stellen durch ein T4-Antigen oder ein Haptoglobinbindungsfragment hievon gebildet werden.

4. Verfahren nach Anspruch 1 oder 2, **dadurch gekennzeichnet, daß** die genannten Stellen oder der gesamte Haptoglobinbindungspartner von einem Antikörper gebildet wird, welcher sich spezifisch an eine α2-Kette eines Haptoglobins vom Phenotyp Hp 2-1 oder Hp 2-2, aber nicht an eine α1-Kette bindet.

5. Verfahren nach Anspruch 4, **dadurch gekennzeichnet, daß** sich der genannte Antikörper spezifisch an ein Epitop bindet, welches aus der folgenden Aminosäuresequenz einer α2-Kette ausgewählt ist: Ala Val Gly Asp Lys Leu Pro Glu Cys Glu Ala Asp Asp Gly Gln Pro Pro Pro Lys Cys Ile und mindestens die folgende Sequenz umfaßt: Glu Ala Asp.

6. Verfahren nach einem der Ansprüche 1 bis 5, **dadurch gekennzeichnet, daß** der genannte Bindungspartner überwiegend aus einem Molekül besteht, welches gegebenenfalls durch chemisches Binden von zwei oder mehr Molekülen hergestellt wird, um so mindestens zwei Haptoglobinbindungsstellen zur Verfügung zu stellen, welches Molekül fähig ist, Haptoglobine verschiedener Phenotypen in unterschiedlichem Ausmaß zu agglutinieren.

7. Verfahren nach Anspruch 6, **dadurch gekennzeichnet, daß** die genannten Bindungsstellen für verschiedene Haptoglobinphenotypen spezifisch sind, um das genannte unterschiedliche Ausmaß an Agglutinierung zu erhalten.

8. Verfahren nach Anspruch 6 oder 7, **dadurch gekennzeichnet, daß** das genannte Molekül eine derartige Größe besitzt, daß das genannte unterschiedliche Ausmaß an Agglutinierung erhalten wird.

9. Verfahren nach einem der Ansprüche 1 bis 5, **dadurch gekennzeichnet, daß** der genannte Bindungspartner Trägerteilchen umfaßt.

10. Verfahren nach Anspruch 9, **dadurch gekennzeichnet, daß** die genannten Trägerteilchen eukaryotische und/oder prokaryotische Zellen oder ein Bruchstück hievon umfassen, welche Rezeptoren tragen, die die genannten Stellen, an welche die Haptoglobine binden können, ausbilden.

11. Verfahren nach Anspruch 10, **dadurch gekennzeichnet, daß** die genannten Zellen Zellen von Streptococcus pyogenes sind, welche insbesondere T4-Antigene als Haptoglobinrezeptoren tragen.

12. Verfahren nach Anspruch 10, **dadurch gekennzeichnet, daß** die genannten Zellen tierische oder menschliche Zellen, insbesondere Leukozyten sind, welche CD-Rezeptoren, insbesondere CD22-Rezeptoren tragen.

13. Verfahren nach Anspruch 9, **dadurch gekennzeichnet, daß** der genannte Bindungspartner Trägerteilchen mit vorzugsweise durch Adsorption und/oder kovalente Bindung daran gebundenen Stellen aufweist, welche Teilchen insbesondere synthetische oder mineralische Teilchen oder natürliche Zellen oder Fragmente hievon umfassen.

14. Verfahren nach Anspruch 13, **dadurch gekennzeichnet, daß** im Fall der spezifischen Bindungsstellen die genannten Trägerteilchen einen Durchmesser von 0,02 µ bis 2 µ und vorzugsweise, im Fall, daß die genannten Bindungsstellen für verschiedene Haptoglobinphenotypen spezifisch sind, von 0,1 µ bis 0,8 µ und im Fall, daß die genannten Bindungsstellen für verschiedene Haptoglobinphenotypen nicht spezifisch sind, von 0,5 µ bis 4 µ, und stärker bevorzugt von 1 µ bis 3 µ besitzen.

15. Verfahren nach Anspruch 13 oder 14, **dadurch gekennzeichnet, daß** die Trägerteilchen bakterielle Antigene, insbesondere T4-Antigene, Haptoglobinantikörper, welche für die verschiedenen Haptoglobinphenotypen spezifisch oder nichtspezifisch sind, und/oder Haptoglobinbindungsfragmente der genannten Antigene oder Antikörper aufweisen, welche daran anhaften, um die genannten Stellen auszubilden, durch welche sich der Bindungspartner an Haptoglobin binden kann.

16. Verfahren nach einem der Ansprüche 1 bis 15, **dadurch gekennzeichnet, daß** die Beziehung zwischen der Konzentration eines Referenzhaptoglobins im biologischen Fluid und dem Ausmaß der Agglutinierung zuerst für mindestens einen Referenzhaptoglobinphenotyp ermittelt wird, insbesondere für den Haptoglobinphenotyp Hp 2-1 und/oder Hp 2-2 und vorzugsweise für den Haptoglobinphenotyp 2-2, und daß der Phenotyp des Haptoglobins im biologischen Fluid durch Vergleichen der Beziehung zwischen der gemessenen Konzentration des Haptoglobins und dem gemessenen Ausmaß an Agglutinierung mit der genannten festgesetzten Beziehung zwischen der Konzentration des Referenzhaptoglobins und dem Ausmaß an Agglutinierung, welche durch das genannte Referenzhaptoglobin erhalten werden, bestimmt wird.

17. Verfahren nach Anspruch 16, **dadurch gekennzeichnet, daß** zur Bestimmung des Phenotyps des Haptoglobins im biologischen Fluid das gemessene Ausmaß an Agglutinierung mittels der genannten Beziehung, welche für das Referenzhaptoglobin zu einer bestimmten Konzentration dieses Referenzhaptoglobins festgesetzt wurde, korreliert wird, wobei das Verhältnis zwischen dieser bestimmten Konzentration und der gemessenen Haptoglobinkonzentration berechnet wird, und basierend auf diesem Verhältnis der Phenotyp des Haptoglobins ermittelt wird, wobei berücksichtigt wird, daß, wenn Haptoglobin Hp 2-2 als Referenzhaptoglobin verwendet wird, das berechnete Verhältnis durchschnittlich nahe 1; 0,5 bzw. 0,125 betragen würde, im Fall, daß das Haptoglobin im biologischen Fluid vom Phenotyp Hp 2-2, Hp 2-1 und Hp 1-1 ist.

18. Verfahren nach einem der Ansprüche 1 bis 17, **dadurch gekennzeichnet, daß** das Ausmaß an Agglutinierung nephelometrisch und/oder turbidimetrisch gemessen wird.

19. Kit zur Bestimmung des Phenotyps eines Haptoglobins in einem biologischen Fluid gemäß dem Verfahren nach Anspruch 1, **dadurch gekennzeichnet, daß** er:
a) einen Haptoglobinbindungspartner, welcher mindestens zwei Stellen aufweist, durch welche dieser an Haptoglobin binden kann, um so fähig zu sein, Haptoglobine zu agglutinieren und/oder dadurch in unterschiedlichem Ausmaß agglutiniert zu werden, in Abhängigkeit vom Haptoglobinphenotyp; und
b) mindestens eine Haptoglobin eines vorbestimmten Phenotyps enthaltende Probe
umfaßt.

20. Kit nach Anspruch 19, **dadurch gekennzeichnet, daß** das Haptoglobin in der genannten Probe vom Phenotyp Hp 2-2 oder Hp 2-1 und vorzugsweise vom Phenotyp Hp 2-2 ist.

21. Kit nach Anspruch 19 oder 20, **dadurch gekennzeichnet, daß** er ferner mindestens ein Reagenz zum Messen der Konzentration von Haptoglobinen umfaßt, insbesondere eine Zubereitung von Antikörpern, welche sich an die verschiedenen Haptoglobinphenotypen binden.

22. Verwendung des Verfahrens nach einem der Ansprüche 1 bis 18 oder des Kits nach einem der Ansprüche 19 bis 21 zur Bewertung eines Arterioskleroserisikos, wobei die Bestimmung eines Haptoglobinphenotyps Hp 2-2 mit einem höheren Arterioskleroserisiko als die Bestimmung eines Haptoglobinphenotyps Hp 1-1 oder 2-1 korreliert ist.

23. Verwendung des Verfahrens nach einem der Ansprüche 1 bis 18 oder des Kits nach einem der Ansprüche 19 bis 21 zur Einschätzung einer auf eine virale Infektion, insbesondere eine HIV-Infektion folgende Prognose eines Patienten, wobei die Bestimmung eines Haptoglobinphenotyps Hp 2-2 mit einer verschlechterten Prognose als die Bestimmung eines Haptoglobinphenotyps Hp 1-1 oder 2-1 korreliert ist.

24. Verwendung des Verfahrens nach einem der Ansprüche 1 bis 18 oder des Kits nach einem der Ansprüche 19 bis 21 zur Bestimmung der Transplantat-Uberlebensrate nach einer Transplantation, insbesondere einer Lebertransplantation, wobei die Bestimmung eines Haptoglobinphenotyps Hp 2-2 mit einer geringeren Überlebensrate als die Bestimmung eines Haptoglobinphenotyps Hp 1-1 oder 2-1 korreliert ist.

25. Verwendung des Verfahrens nach einem der Ansprüche 1 bis 18 oder des Kits nach einem der Ansprüche 19 bis 21 zur Verbesserung der Interpretation von Laborparametern, welche von der Gruppe bestehend aus Serumhaptoglobinkonzentration, Plasmalipide, insbesondere Gesamtlipide und LDL-Cholesterin, Plasmaproteine, insbesondere Ferritin, Immunoglobulin A und Ceruloplasmin, und andere biochemische Parameter, wie Serumeisen, Transferrinsättigung, Vitamin C und Vitamin E ausgewählt sind, wobei die Bestimmung eines Haptoglobinphenotyps Hp 2-2 mit anderen Referenzwerten als die Bestimmung eines Haptoglobinphenotyps Hp 1-1 oder 2-1 korreliert ist.

26. Verwendung eines Haptoglobinbindungspartners, welcher mindestens zwei Stellen aufweist, durch welche er sich an Haptoglobin binden kann, um so fähig zu sein, Haptoglobine zu agglutinieren und/oder dadurch in unterschiedlichem Ausmaß agglutiniert zu werden, in Abhängigkeit vom Haptoglobinphenotyp, zur Bestimmung des Phenotyps eines Haptoglobins in einem biologischen Fluid, insbesondere in Blut, Plasma, Serum, Liquor, Urin, Zellextrakt oder Gewebeextrakt.

27. Verwendung nach Anspruch 26, **dadurch gekennzeichnet, daß** die genannten Stellen am Haptoglobinbindungspartner durch einen Peptid, einen monoklonalen oder polyklonalen Antikörper, ein F_{ab}-, F_{ab'}- oder F(ab')₂-Fragment eines Antikörpers, ein Lectin, einen Zellrezeptor, einen molekularen Imprint eines Haptoglobins, ein bakterielles Antigen und/oder Fragmente hievon, welche die jeweilige Haptoglobinbindungsstelle enthalten, gebildet werden.

28. Verwendung nach Anspruch 26 oder 27, **dadurch gekennzeichnet, daß** der Haptoglobinbindungspartner bakterielle Zellen, insbesondere Zellen von T4-Antigene tragendem Streptococcus pyogenes oder Fraktionen hievon, welche Haptoglobinbindungsstellen enthalten, umfaßt.

29. Haptoglobinbindungspartner zur Verwendung in einem Verfahren nach einem der Ansprüche 1 bis 18 und/oder im Kit nach einem der Ansprüche 19 bis 21, **dadurch gekennzeichnet, daß** er von den gesamten Zellen von Streptococcus pyogenes verschieden ist und mindestens zwei Stellen aufweist, durch welche er an Haptoglobin binden kann, um so fähig zu sein, Haptoglobine zu agglutinieren und/oder dadurch in unterschiedlichem Ausmaß agglutiniert zu werden, in Abhängigkeit vom Haptoglobinphenotyp.

30. Haptoglobinbindungspartner nach Anspruch 29, **dadurch gekennzeichnet, daß** er Trägerteilchen umfaßt, welche die genannten Stellen daran gebunden enthalten, vorzugsweise durch Adsorption und/oder kovalente Bindung.

31. Haptoglobinbindungspartner nach Anspruch 29 oder 30, **dadurch gekennzeichnet, daß** er einen Antikörper oder ein Antikörperfragment umfaßt, welches an eine α2-Kette eines Haptoglobins vom Phenotyp Hp 2-1 oder Hp 2-2, aber nicht an eine α1-Kette bindet.

32. Kit zur Bestimmung des Phenotyps eines Haptoglobins in einem biologischen Fluid, **dadurch gekennzeichnet, daß** er einen wie in einem der Ansprüche 29 bis 31 definierten Haptoglobinbindungspartner umfaßt.

## Revendications

1. Procédé pour la détermination du phénotype d'une haptoglobine dans un liquide biologique, **caractérisé en ce qu'**il comprend les étapes suivantes :
a) mise en contact d'un échantillon du liquide biologique avec un partenaire de liaison à l'haptoglobine qui comporte au moins deux sites lui permettant de se fixer à l'haptoglobine, de manière à pouvoir agglutiner les haptoglobines et/ou à être agglutiné par celles-ci à différents degrés, en fonction du phénotype d'haptoglobine, et mesure du degré d'agglutination;
b) mesure de la concentration de l'haptoglobine dans le liquide biologique ; et
c) détermination, sur la base du degré d'agglutination et de la concentration d'haptoglobine, du phénotype de l'haptoglobine dans le liquide biologique.

2. Procédé selon la revendication 1, **caractérisé en ce que** les sites par lesquels ledit partenaire de liaison peut se lier aux haptoglobines, sont constitués par un peptide, un anticorps monoclonal ou polyclonal, un fragment F_{ab}, F_{ab'} ou F(ab')₂ d'un l'anticorps, une lectine, un récepteur cellulaire, une empreinte moléculaire d'une haptoglobine, un antigène bactérien et/ou des fragments de ceux-ci contenant le site de liaison à l'haptoglobine respective.

3. Procédé selon la revendication 2, **caractérisé en ce que** lesdits sites sont constitués par un antigène T4 ou un fragment de celui-ci se liant à l'haptoglobine.

4. Procédé selon la revendication 1 ou 2, **caractérisé en ce que** lesdits sites ou le partenaire complet de liaison à l'haptoglobine est(sont) constitué(s) par un anticorps qui se lie spécifiquement à une chaîne α2 d'une haptoglobine du phénotype Hp 2-1 ou Hp 2-2 mais non à une chaîne α1.

5. Procédé selon la revendication 4, **caractérisé en ce que** ledit anticorps se lie spécifiquement à un épitope choisi dans la séquence d'aminoacides suivante d'une chaîne α2 : ala val gly asp lys leu pro glu cys glu ala asp asp gly gin pro pro pro lys cys ile et comprenant au moins la séquence suivante : glu ala asp.

6. Procédé selon l'une quelconque des revendications 1 à 5, **caractérisé en ce que** ledit partenaire de liaison consiste principalement en une molécule, éventuellement produite par liaison chimique de deux molécules ou plus, de manière à fournir lesdits au moins deux sites de liaison à l'haptoglobine, laquelle molécule est capable d'agglutiner à différents degrés des haptoglobines de différents phénotypes.

7. Procédé selon la revendication 6, **caractérisé en ce que** lesdits sites de liaison sont spécifiques de différents phénotypes d'haptoglobines, pour l'obtention dudit différent degré d'agglutination.

8. Procédé selon la revendication 6 ou 7, **caractérisé en ce que** ladite molécule a une taille telle qu'on obtient lesdits différents degrés d'agglutination.

9. Procédé selon l'une quelconque des revendications 1 à 5, **caractérisé en ce que** ledit partenaire de liaison comprend des particules porteuses.

10. Procédé selon la revendication 9, **caractérisé en ce que** lesdites particules porteuses comprennent des cellules eucaryotes et/ou procaryotes ou une fraction de celles-ci portant des récepteurs formant lesdits sites auxquels peuvent se lier les haptoglobines.

11. Procédé selon la revendication 10 **caractérisé en ce que** lesdites cellules sont des cellules de *Streptococcus pyogenes* portant en particulier des antigènes T4 en tant que récepteurs d'haptoglobînes.

12. Procédé selon la revendication 10, **caractérisé en ce que** lesdites cellules sont des cellules humaines ou animales, en particulier des leucocytes, portant des récepteurs CD, en particulier des récepteurs CD22.

13. Procédé selon la revendlcatlon 9, **caractérisé en ce que** ledit partenaire de liaison comprend des particules porteuses comportant lesdits sites attachés à celles-ci, de préférence par adsorption et/ou liaison covalente, lesdites particules comprenant en particulier des particules minérales ou synthétiques ou des cellules naturelles ou des fragments de celles-ci.

14. Procédé selon la revendication 13, **caractérisé en ce que**, dans le cas où les sites de liaison sont spécifiques, lesdites particules porteuses ont un diamètre compris entre 0,02 et 2 µm, et, de préférence, dans le cas où lesdits sites de liaison sont spécifiques de différents phénotypes d'haptoglobines, entre 0,1 et 0,8 µm, et, dans le cas lesdits sites de liaison ne sont pas spécifiques de différents phénotypes d'haptoglobines, entre 0,5 µm et 4 µm, et encore mieux entre 1 µm et 3 µm.

15. Procédé selon la revendication 13 ou 14, **caractérisé en ce que** les particules porteuses sont des antigènes bactériens, en particulier les antigènes T4, des anticorps anti-haptoglobine qui sont spécifiques ou non des différents phénotypes d'haptoglobines et/ou des fragments de liaison à l'haptoglobine desdits antigènes ou anticorps adhérant à ceux-ci, pour constituer lesdits sites par lesquels le partenaire de liaison peut se lier à l'haptoglobine.

16. Procédé selon l'une quelconque des revendications 1 à 15, **caractérisé en ce qu'**on établit d'abord la relation entre la concentration d'une haptoglobine de référence dans le liquide biologique et le degré d'agglutination, pour au moins un phénotype d'haptoglobine de référence, en particulier pour le phénotype d'haptoglobine Hp 2-1 et/ou Hp 2-2, et de préférence pour le phénotype d'haptoglobine Hp 2-2, et on détermine le phénotype de l'haptoglobine dans le liquide biologique en comparant la relation entre la concentration mesurée de l'haptoglobine et le degré mesuré d'agglutination, avec ladite relation établie entre la concentration de l'haptoglobine de référence et le degré d'agglutination obtenu par ladite haptoglobine de référence.

17. Procédé selon la revendication 16, **caractérisé en ce que**, pour déterminer le phénotype de l'haptoglobine dans le liquide biologique, on met le degré mesuré d'agglutination en corrélation, au moyen de ladite relation établie pour l'haptoglobine de référence, avec une concentration particulière de cette haptoglobine de référence, on calcule le rapport entre cette concentration particulière et la concentration mesurée d'haptoglobine, et on détermine le phénotype de l'haptoglobine sur la: base de ce rapport, en tenant compte qu'en prenant comme haptoglobine de référence l'haptoglobine Hp 2-2, le rapport calculé serait en moyenne voisin, de 1, 05 et 0,125, respectivement, lorsque l'haptoglobine dans le liquide biologique est du phénotype Hp 2-2, Hp 2-1 et Hp 1-1.

18. Procédé selon l'une quelconque des revendications 1 à 17, **caractérisé en ce que** le degré d'agglutination est mesuré par néphélométrie et/ou turbidimétrie.

19. Nécessaire pour la détermination du phénotype d'une haptoglobine dans un liquide biologique, selon le procédé de la revendication 1, **caractérisé en ce qu'**il comprend :
a) un partenaire de liaison à l'haptoglobine qui comporte au moins deux sites par lesquels il peut se lier à l'haptoglobine, de manière à pouvoir agglutiner des haptoglobines et/ou à être agglutiné par celles-ci à différents degrés, en fonction du phénotype de l'haptoglobine ; et
b) au moins un échantillon comprenant une haptoglobine d'un phénotype préétabli.

20. Nécessaire selon la revendication 19, **caractérisé en ce que** l'haptoglobine dans ledit échantillon est du phénotype Hp 2-2 ou Hp 2-1, et de préférence du phénotype Hp 2-2.

21. Nécessaire selon la revendication 19 ou 20, **caractérisé en ce qu'**il comprend en outre au moins un réactif pour mesurer la concentration d'haptoglobines, en particulier une préparation d'anticorps qui se lie aux différents phénotypes d'haptoglobines.

22. Utilisation du procédé selon l'une quelconque des revendications 1 à 18, ou du nécessaire selon l'une quelconque des revendications 19 à 21, pour l'évaluation du risque athéroscléreux, la constatation d'un phénotype d'haptoglobine Hp 2-2 étant en corrélation avec un plus grand risque athéroscléreux que la constatation d'un phénotype d'haptoglobine Hp 1-1 ou 2-1.

23. Utilisation du procédé selon l'une quelconque des revendications 1 à 18 ou du nécessaire selon l'une quelconque des revendications 19 à 21, pour l'estimation du pronostic d'un patient après une infection virale, en particulier une infection à VIH, la constatation d'un phénotype d'haptoglobine Hp 2-2 étant en corrélation avec un moins bon pronostic que la constatation d'un phénotype d'haptoglobine Hp 1-1 ou 2-1.

24. Utilisation du procédé selon l'une quelconque des revendications 1 à 18 ou du nécessaire selon l'une quelconque des revendications 19 à 21, pour la détermination de la survie d'un greffon après transplantation, en particulier une transplantation du foie, la constatation d'un phénotype d'haptoglobine Hp 2-2 étant en corrélation avec une plus faible survie que la constatation d'un phénotype d'haptoglobine Hp1-1 ou 2-1.

25. Utilisation du procédé selon l'une quelconque des revendications 1 à 18 ou du nécessaire selon l'une quelconque des revendications 19 à 21, pour améliorer l'interprétation de paramètres de laboratoire choisis dans l'ensemble comprenant la concentration sérique d'haptoglobine, des lipides plasmatiques, en particulier des lipides totaux et du LDL-cholestérol, de protéines plasmatiques, en particulier la ferritine, l'immunoglobuline A et la céruloplasmine, et d'autres paramètres biochimiques tels que fer sérique, la saturation de trans-ferrine, la vitamine C et la vitamine E, la constatation d'un phénotype d'haptoglobine Hp 2-2 étant en corrélation avec d'autres valeurs de référence que la détermination d'un phénotype d'haptoglobine Hp 1-1 ou 2-1.

26. Utilisation d'un partenaire de liaison à l'haptoglobine, qui comporte au moins deux sites par lesquels il peut se fixer à l'haptoglobine, pour pouvoir agglutiner des haptoglobines et/ou être agglutiné par celles-ci à différents degrés, en fonction du phénotype de l'haptoglobine, pour la détermination du phénotype d'une haptoglobine dans un liquide biologique, en particulier dans du sang, du plasma, du sérum, du liquide céphalo-rachidien, de l'urine, un extrait cellulaire ou un extrait tissulaire.

27. Utilisation selon la revendication 26, **caractérisée en ce que** lesdits sites sur le partenaire de liaison à l'haptoglobine sont constitués par un peptide, un anticorps monoclonal ou polyclonal, un fragment F_{ab}, F_{ab'} ou F(ab')₂ d'un anticorps, une lectine, un récepteur cellulaire, une empreinte moléculaire d'une haptoglobine, un antigène bactérien et/ou des fragments de ceux-ci contenant le site de liaison à l'haptoglobine respective.

28. Utilisation selon la revendication 26 ou 27, **caractérisée en ce que** le partenaire de liaison à l'haptoglobine comprend des cellules bactériennes, en particulier des cellules de *Streptococcus pyogenes* portant des antigènes T4, ou des fractions de celles-ci contenant des sites, de liaison à l'haptoglobine.

29. Partenaire de liaison à l'haptoglobine pour l'utilisation dans un procédé selon l'une quelconque des revendications 1 à 18 et/ou dans le nécessaire selon l'une quelconque des revendications 19 à 21, **caractérisé en ce qu'**il est différent de cellules entières de *Streptococcus pyogenes* et comporte au moins deux sites par lesquels il peut se lier à une haptoglobine de manière à pouvoir agglutiner des haptoglobines et/ou à être agglutiné par celles-ci à différents degrés, en fonction du phénotype de l'haptoglobine.

30. Partenaire de liaison à l'haptoglobine selon la revendication 29, **caractérisé en ce qu'**il comprend des particules porteuses comportant lesdits sites attachés à celles-ci, de préférence par adsorption et/ou liaison covalente,

31. Partenaire de liaison à l'haptoglobine selon la revendication 29 ou 30, **caractérisé en ce qu'**il comprend un anticorps ou un fragment d'anticorps qui se lie spécifiquement à une chaîne α2 d'une haptoglobine du phénotype Hp 2-1 ou Hp 2-2, mais non à une chaîne α1.

32. Nécessaire pour la détermination du phénotype d'une haptoglobine dans un liquide biologique, **caractérisé en ce qu'**il comprend un partenaire de liaison à l'haptoglobine tel que défini dans l'une quelconque des revendications 29 à 31.
